# EUROPEAN PATENT APPLICATION

(11) **EP 2 667 237 A1**
(43) Date of publication of application: **27.11.2013**
(21) Application number: 12754754.5
(22) Date of filing: 02.03.2012
(51) Int. Cl.: G02B 27/01, A61B 3/00, A61H 5/00, A61F 9/00

(54) **EQUIPMENT AND METHOD FOR EXAMINING, DIAGNOSING OR AIDING THE DIAGNOSIS, AND THERAPY OF FUNCTIONAL VISION PROBLEMS**

(30) Priority: 04.03.2011 ES 201100247 P
(71) Applicant: Davalor Consultoría Estratégica y Tecnológica S.L., 31620 Gorraiz, Navarra (ES)
(72) Inventor: MARCOS MUÑOZ, Juan José, 31620 Gorraiz, Navarra (ES)
(74) Representative: Cabinet Plasseraud
(86) International application number: PCT/ES2012/000051
(87) International publication number: WO 2012/120164

(57) **Abstract**

The present invention relates to an equipment and a method for examining, diagnosing or aiding the diagnosis and therapy of functional vision problems formed by an apparatus including respective functional assemblies (2) intended to be placed in front of the user's eyes, each functional assembly (2) comprising a display screen (5), lens elements (6) focusing images of the display screen (5) at required distances, an image capturing camera (7) and light projecting sources (8), which functional assemblies (2) are connected with an electronic unit (10) with which a computer (11), an external variable detection system (12-13), an audio communication system (14-15) and a control element (16) are in turn connected for determining functional vision problems and performing exercises for correcting said problems.

## Description

### Field of the Art

The present invention relates to examining, diagnosing or aiding vision diagnosis and therapy, proposing equipment and a method which allows performing said functions practically.

### State of the Art

95% of information is received through the eyes, therefore, the eyes are the most important way for spatial orientation, emotional communication, and particularly for learning (for example, in relation to formal recognition skills, reading skills, reading comprehension skills, etc.), therefore it is convenient to perform a periodic eye examination to detect and treat functional problems that one may have.

Apparatuses for testing vision which allow checking the condition of the eyes and the functional capacity of eyesight are known, but the existing apparatuses are generally simple, requiring the involvement of experts for evaluating patients' eye performance, such that given the shortage of professionals dedicated to serving the entire population, vision examinations cannot be conducted on people often enough.

### Object of the Invention

The invention proposes equipment and a method which allow examining, diagnosing or aiding the diagnosis and therapy of functional vision problems, with simple means which can be handled by the user himself or by a professional.

The equipment comprises a spectacle-like device, which includes an image display screen, lens elements, one or more (visible or non-visible) light sources and an image capturing camera in the corresponding area in front of each eye, all of them being located in a casing determining a free space in front of the eye of application, and connected with an independent electronic unit which is in turn connected with a computer, with an audio communication system and with external light sensors and sensors for sensing the position of the user's head.

Said assembly allows transmitting images by means of the electronic unit to the screen located in front of the eye, said images being seen by the eye through the lens elements, while at the same time the eye receives a light projection emitted by the light sources, whereby the eye produces reflections which are captured by the camera, signals being transmitted depending on the reflections to the electronic unit which combines said signals with those of the external sensors, generating data which is transmitted to the computer to be processed.

Therefore, an equipment exists which allows obtaining functional parameters of the eyes in real time, such as the movement, the point of convergence of a gaze, the pupil diameter, the distance of the focal plane and the lens curvature, for comparing this data with the pre-established patterns defining normal vision, thus determining the functional vision problems of the eye, for the purpose of adopting a therapy which allows reducing or eliminating those functional vision problems of the analyzed eye.

Through the electronic unit and the audio or image transmission system, the user can also interact with the equipment for determining actions which allow obtaining data of the specific parameters to be measured.

The casing containing the functional elements which are arranged in front of the eyes allows using the equipment over the spectacles of the user if he/she had any, whereby the data obtained on the vision are completely real in relation to the particular circumstances of the eyes of each individual.

On the other hand, for obtaining data on the different parameters of the eyes, the use of a set of exchangeable lens elements which can be replaced manually or by means of an automatic loader suitable for that purpose, is envisaged.

According to a practical embodiment of the invention, the casing of each functional assembly has an opening for the selective insertion of cartridges having the lens elements. Therefore, depending on the test to which the user's eyes are to be subjected, the cartridge with the most suitable lens elements can be selected.

Therefore, said equipment object of the invention has very advantageous features for detecting functional vision problems, acquiring its own identity and preferred character for said function.

### Description of the Drawings

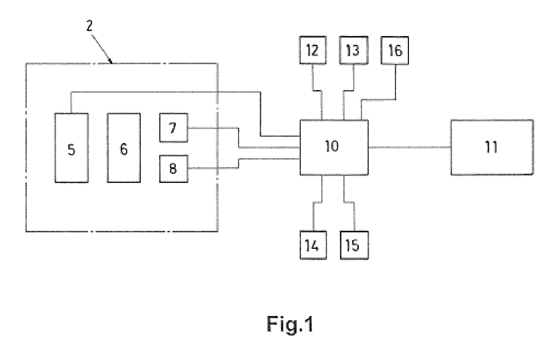

Figure 1 shows a block diagram of the assembly making up the equipment object of the invention for examining the sight of one eye.

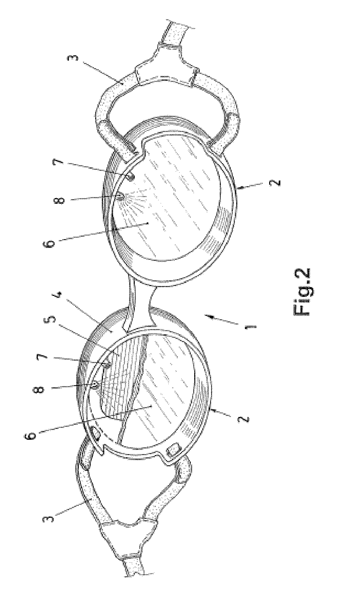

Figure 2 is a schematic perspective view of a practical embodiment of a spectacle-like apparatus provided with respective assemblies made according to the invention, for the application over a user's eyes.

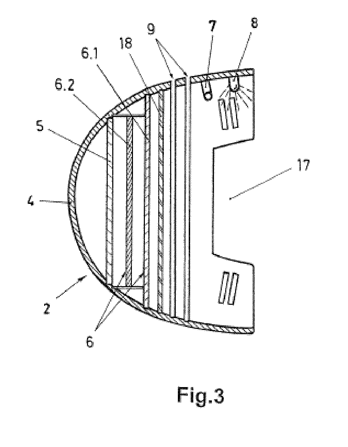

Figure 3 is a schematic cross-section view of one of the partial assemblies of the apparatus of the preceding figure.
Figure 4 shows a schematic perspective view of another practical embodiment of equipment according to the invention, wherein cartridges with lens elements are in an insertion position with respect to their respective housing casings.
Figure 5 shows a schematic cross-section view of an assembly formed by a cartridge inserted in its respective casing according to the embodiment of the preceding figure.
Figure 6 is a schematic sectioned view of the disposition of a cartridge in a position facing the insertion in a housing casing.
Figure 7 shows a schematic sectioned view of the assembly of the preceding figure with the cartridge inserted and laterally tilted inside the housing casing.
Figure 8 shows a schematic view of the equipment of Figure 4 provided with securing means and incorporated on the head of a user.
Figure 9 shows in a detail partial perspective view of the equipment, one of the functional assemblies with a side opening in an open position being shown.
Figure 10 shows the same detailed view of the preceding figure with the side opening in a closed position.
Figure 11 shows another practical embodiment of the equipment of the invention on the head of a user.
Figure 12 is a detail partial enlarged view of the preceding figure.

### Detailed Description of the Invention

The object of the invention relates to equipment and a method for examining eyesight for the purpose of detecting functional vision defects in healthy eyes to enable adopting a therapy for correcting said defects.

In a practical non-limiting embodiment, the proposed equipment is envisaged to be formed by a spectacle apparatus (1), made up of functional assemblies (2) intended for application over each of the eyes, said apparatus (1) being provided with securing means (3) which can be of any type to establish a fixed fastening on the user's head.

Each of the functional assemblies (2) intended for conducting the eye examination comprises a casing (4), inside which there is arranged a display screen (5), and in front of the display there is arranged a set of lens elements (6) as well as an image capturing camera (7) and one or more light projecting sources (8).

The lens elements (6) in said functional assembly (2) can be optical lenses or another type of element performing a similar function, for example, opaque discs with several holes or with a central slot, according to one embodiment one of said elements (6.1) being arranged in a fixed manner in the casing (4) and another element (6.2) in a mobile disposition with the possibility of moving closer to and away from said fixed element (6.1), allowing determining a variable focal length, like a zoom, whereas other elements (not depicted) are inserted in the slots (9) of the casing (4), being able to be replaced manually or by means of an automatic loader suitable to that end.

On the other hand, the assembly of the equipment is connected with an electronic unit (10) controlling the operational actions of the functional assemblies (2) and through which the user or another person can interact with the system for determining the operating modes in relation to vision examinations to be performed on the eyes of application.

The electronic unit (10) is connected with a computer (11), and an external variable detection system and an audio communication system, comprising the external variable detection system, for example, one or more sensors (12) for sensing the position of the user's head and a sensor (13) for sensing ambient light are further connected therewith, whereas the audio communication system comprises headphones (14) and a microphone (15). A control element (16) is also provided in relation to the electronic unit (10), by means of which the user can interact with the operating system of the functional assemblies (2) to perform the desired operations.

Therefore, to use the equipment, the apparatus (1) is secured on the user's head with the functional assemblies (2) in a disposition in front of the eyes, images being sent by means of the electronic unit (10) to the screen (5), such that said images are seen by the corresponding eye through the lens elements (6), while at the same a ray of light is projected on the eye by means of the sources (8).

Hence, when the eye sees the images appearing on the screen (5), it reflects the light projected thereon, said reflections being captured together with the image of the eye by the camera (7), which transmits signals to the electronic unit (10) depending on the reflections, wherein said signals are combined with those provided by the external sensors (12-13), generating data corresponding to parameters of the examined eye, which is transmitted to the computer (11) to be processed.

Data corresponding to different parameters of the eyes, such as the movement, the point of convergence of a gaze, the pupil diameter, the distance of the focal plane and the lens curvature can thus be obtained in the action of the eyes upon viewing the images appearing on the screens (5), said data being compared with the pre-established patterns of a normal vision for determining functional vision anomalies, such that by means of projecting images on the screens (5) the eyes are subjected to exercises which allow improving the functional problems detected.

The functional assemblies (2) are determined such that when in use, there is a free space between said functional assemblies (2) and the user's eyes which allows the user to use the equipment while wearing his usual spectacles, if he/she has any, whereby the results obtained from the vision examination are completely real in relation to the eyes of application.

The casing (4) of the functional assemblies (2) is also provided with side openings (17) through which the ambient light can enter from the outside and the user can see the environment he/she is with his peripheral vision.

In the apparatus (1) the functional assemblies (2) incorporating the elements for vision examination are provided with a movement and orientation system (manual or automatic), for adjusting the position of said functional assemblies (2) depending on the interpupillary distance and on the plane perpendicular to the direction of vision of the eyes of each user, so that the results of vision examination are correct in each case.

On the other hand, the functional assemblies (2) in the apparatus (1) can be structurally independent, each of them integrated in a respective casing (4), but similarly, without it altering the concept of the invention, the two functional assemblies (2) can be integrated in a single common casing (4), a single strategically incorporated image capturing camera (7) being able to be provided for working in relation to both functional assemblies (2).

The integration of the functional assemblies (2) in the housing casing or casings (4) is established with a hermetic closure by means of a transparent element (18) to prevent the entry of dust which may affect the operating components causing their deterioration.

The slots (9) for inserting the replaceable lens elements (6) are further provided with brush-like hair strips to prevent in turn the entry of dust through said slots (9).

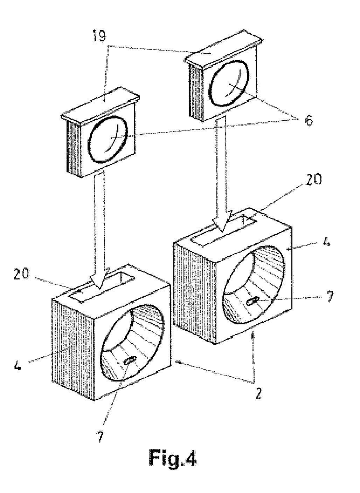

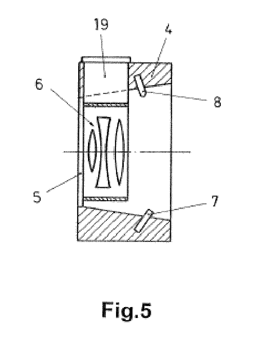

Figure 4 shows another practical embodiment of the equipment object of the invention, wherein the casing (4) of each functional assembly (2) has an opening (20) for the selective insertion of one or more cartridges (19) having the lens elements (6). Therefore, depending on the test to which the user's eyes are to be subjected, the cartridge (19) with the most suitable lens elements (6) can be selected. The sectioned view of Figure 5 shows the cartridge (19) inserted in the corresponding casing (4), being arranged in front of the respective display screen (5).

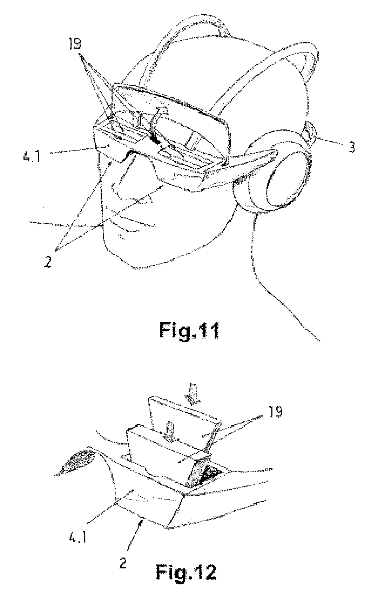

In a preferred solution such as that depicted in Figure 11, the cartridges (19) having the lens elements (6) are able to be inserted through openings defined in an outer casing (4.1), such that said outer casing (4.1) covers both the cartridges (19) and the casings (4) of the functional assemblies (2) in which the display screens (5), the image capturing cameras (7) and the light projecting sources (8) are arranged.

As can be seen in Figure 12, one or more cartridges (19) which allow introducing additional optomechatronics to the equipment can be inserted in series in the openings defined in the outer casing (4.1). Therefore, the first cartridge closest to the eye can contain the optomechatronics of the lens elements (6) allowing the user to see the display screen of the display and the equipment to show the display screen (5) at any apparent distance of the user (for example, by means of a monofocal lens that can be moved axially or a multifocal lens), whereas the other cartridges can contain, for example, the optomechatronics for correcting emmetropia and/or astigmatism of the eye, or others.

In order for the images projected on the display screen (5) to be stereoscopically and realistically seen by the user's eyes, it has been provided that the images depicted in each display screen (5) are geometrically out-of-phase depending on the perspective of each eye, and that the lens elements (6) can change their focal length depending on the area of the image which is being seen by the user's eyes.

The lens elements (6) can be lenses or lens assemblies of any type which allows focusing images of the display screen (5) at variable distances, being able to be, for example, electro-optical lenses which can be electrically deformed (multifocal lenses), or several optical lenses which can be moved axially with respect to one another (mobile fixed focus lenses), or combinations thereof.

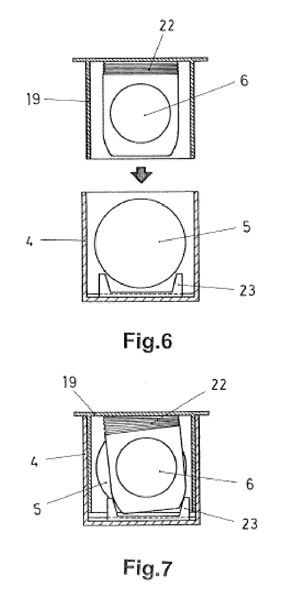

To align the lens elements (6) with respect to each eye, as seen in Figures 6 and 7, the lens elements (6) are attached to the casing (4) of the cartridges (19) by means of elastic elements (22) which allows tilting said lens elements (6) to locate them in the required position. The elastic elements (22) can have different embodiments, therefore they can be a bellows arranged in the upper part of the cartridges (19) as depicted in drawings, a circumferential bellows, several helical springs in different directions or other similar embodiments.

As can be seen in detail in Figure 7, according to one embodiment, the lens elements (6) are tilted to the position required inside the housing casing (4) by means of a positioning carriage (23) which is assembled on a transverse guide inside the casings (4), said carriage (23) being able to be driven manually or by means of automatic elements. Therefore, once a cartridge (19) is correctly inserted in the opening (20) of the respective casing (4), the lower part of the cartridge (20) is anchored in the carriage (23) such that, by moving said carriage (23) laterally, the elastic elements (22) of the lens element (6) bend allowing said lens element (6) to be aligned in the required position in front of the corresponding eye of the user.

According to a preferred embodiment, instead of tilting the lens elements (6) by means of a carriage (23), formations by way of spears with a predefined structure have been provided inside the casings (4) and integral therewith.

Therefore, upon inserting the cartridge (19) in its housing casing (4), the lens element (6) which is incorporated in the cartridge (19) encounters an obstacle which is the spear, which spear forces said lens element (6) to move laterally and/or vertically, which it will be able to do when the elastic elements (22) securing it are deformed. If the spears and the contact area of same on the lens element (6) are provided with a suitable geometry, said lens elements (6) will be aligned in a completely automatic and passive manner.

In this case, for adjusting the casings (4) depending on the interpupillary distance and on the direction of vision of the eyes of each user, said casings (4) are able to be moved vertically and/or horizontally.

If the casings (4) do not comprise the cartridges (19), as is the case of Figure 11, the functional assemblies (2) or the cartridges (19) themselves can have means for moving vertically or horizontally, and therefore arranging the lens elements (6) depending on the interpupillary distance and on the direction of vision of the eyes of the user who is using the equipment.

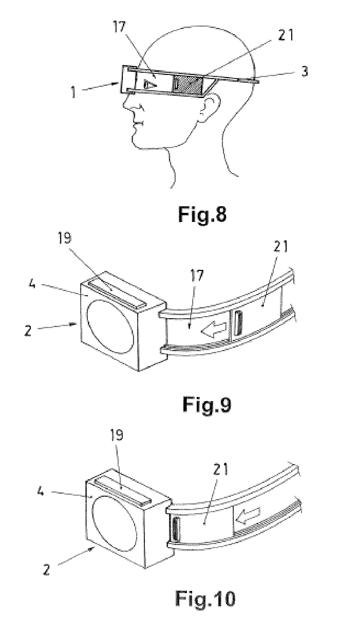

The apparatus (1) applied on a user's eyes, fastened with the means (3) establishing a fixed fastening on the head can be seen in Figure 8. The side openings (17) through which the user can see the environment surrounding him with his peripheral vision being defined in relation to the casings (4) of the functional assemblies (2).

To allow the selective blocking of the side openings (17), when necessary, depending on the tests to be conducted, practicable closure gates (21) in relation to said side openings (17) have been provided, such that, as seen in Figure 9, in a normal position said gates (21) are moved leaving the respective side openings (17) free, and when required by the diagnosis conditions, as seen in Figure 10, the gates (21) can be moved to block the side openings (17).

According to one embodiment, closures (not depicted) made of electro-optical material susceptible to transparency variation are arranged in relation to the side openings (17), which closures allow selectively varying the passage of light through said side openings (17). In this case the closures are arranged in a fixed manner in the side openings (17) and, by means of varying the opacity of said closures, the passage of light into the equipment can be selectively controlled.

Therefore, for the practical use of the equipment, images are shown on the display screens (5) to the user, and parameters of the user's eyes while viewing said images are calculated, which parameters are compared with pre-established patterns for determining functional vision problems. Therefore, by means of projecting images on the screens (5) the eyes are subjected to exercises allowing improving the functional problems detected.

For treatment of the functional problems detected to be efficient, it is essential that the ocular neuromotor system of the user is in a virtual environment suitably representing the three-dimensional perception and the distance perception with respect to the perception of the actual equivalent environment.

To achieve this, the images can be projected in the virtual space stereoscopically (three-dimensionally) and at different distances from the user by means of the display screens (5) and the lens elements (6).

To that end, the image of the user's eyes is first captured by means of the cameras (7) and the direction of gaze of each eye, the point of convergence of a user's gaze and the distance of the point of convergence to the user are calculated from same in the electronic unit (10), controlling the lens elements (6) so that they adapt their focal length according to the actual depth corresponding to the distance of the point of convergence of the user's gaze to the user.

Therefore, each image projected on the display screens (5) is encoded according to actual depths, being able to assign for example, by means of the electronic unit (10), a specific blurriness, which for example is zero blurriness in the proximity of the focal plane of the user (which is that containing the point of convergence of the lines of gaze of a user's eyes) and progressively greater blurriness in increasingly further planes, to the contents of each plane of the three-dimensional image depicted on the plane of the display according to its virtual depth or distance to the user.

## Claims

1. An equipment for examining, diagnosing or aiding the diagnosis and therapy of functional vision problems, formed by a spectacle apparatus (1) provided with two functional assemblies (2) intended to be placed in front of the user's eyes, **characterized in that** each functional assembly (2) comprises a casing (4), inside which there is arranged a display screen (5), lens elements (6), an image capturing camera (7) and one or more light projecting sources (8), the functional assemblies (2) being connected with an electronic unit (10), which is in turn connected with a computer (11), whereas an external variable detection system including the detection of the position of the user's head and the detection of environmental variables, an audio communication system and a control element (16) which allows interacting with the functional assemblies (2) are connected with said electronic unit (10) for measuring the parameters of eye performance when viewing images shown on the screen (5) of said functional assemblies (2).

2. The equipment for examining, diagnosing or aiding the diagnosis and therapy of functional vision problems according to claim 1, **characterized in that** the lens elements (6) comprise an element (6.1) arranged in a fixed manner in the casing (4) and a mobile element (6.2) which can be moved closer to and away from the fixed element (6.1).

3. The equipment for examining, diagnosing or aiding the diagnosis and therapy of functional vision problems according to claim 1, **characterized in that** the lens elements (6) comprise elements which are inserted in slots (9) of the casing (4), being able to be replaced manually or with an automatic loader.

4. The equipment for examining, diagnosing or aiding the diagnosis and therapy of functional vision problems according to claim 1, **characterized in that** the external variable detection system comprises one or more sensors (12) for sensing the position of the user's head and a sensor (13) for sensing ambient light.

5. The equipment for examining, diagnosing or aiding the diagnosis and therapy of functional vision problems according to claim 1, **characterized in that** the audio communication system comprises headphones (14) and a microphone (15), by means of which the user can communicate with the functional assemblies (2) through the electronic unit (10) .

6. The equipment for examining, diagnosing or aiding the diagnosis and therapy of functional vision problems according to claim 1, **characterized in that** the light projecting sources (8) emit visible or non-visible light.

7. The equipment for examining, diagnosing or aiding the diagnosis and therapy of functional vision problems according to claim 1, **characterized in that** the functional assemblies (2) are arranged in a disposition which allows moving and orientating them manually or automatically for adjusting said functional assemblies (2) depending on the interpupillary distance and on the direction of vision of the eyes of each user.

8. The equipment for examining, diagnosing or aiding the diagnosis and therapy of functional vision problems according to claim 1, **characterized in that** the apparatus (1) comprising the functional assemblies (2) has securing means (3) to establish a fixed fastening on the user's head.

9. The equipment for examining, diagnosing or aiding the diagnosis and therapy of functional vision problems according to claim 1, **characterized in that** the casing (4) of the functional assemblies (2) has side openings (17) through which the ambient light enters from the outside and the user can see the environment he/she is in.

10. The equipment for examining, diagnosing or aiding the diagnosis and therapy of functional vision problems according to claim 1, **characterized in that** the functional assemblies (2) are determined such that when in use, there is a space between said functional assemblies (2) and the user's eyes allowing the user to wear his usual spectacles, if he/she has any.

11. The equipment for examining, diagnosing or aiding the diagnosis and therapy of functional vision problems according to claim 1, **characterized in that** the integration of functional assemblies (2) in the housing casing (4) is established with a hermetic closure by means of a transparent element preventing the entry of dust.

12. The equipment for examining, diagnosing or aiding the diagnosis and therapy of functional vision problems according to claim 1, **characterized in that** the functional assemblies (2) are integrated in a common casing (4).

13. The equipment for examining, diagnosing or aiding the diagnosis and therapy of functional vision problems according to claim 1, **characterized in that** in relation to the functional assemblies (2) there is arranged an image capturing camera (7) strategically incorporated to work with respect to both functional assemblies (2).

14. The equipment for examining, diagnosing or aiding the diagnosis and therapy of functional vision problems according to claim 1, **characterized in that** the lens elements (6) are incorporated in cartridges (19) which are able to be inserted through an opening (20) defined in the casing (4) of each functional assembly (2).

15. The equipment for examining, diagnosing or aiding the diagnosis and therapy of functional vision problems according to claim 1, **characterized in that** the lens elements (6) are incorporated in cartridges (19) which are able to be inserted through openings defined in an outer casing (4.1), which in turn comprises the casings (4) of the functional assemblies (2).

16. The equipment for examining, diagnosing or aiding the diagnosis and therapy of functional vision problems according to claim 1, **characterized in that** the lens elements (6) are formed by electro-optical lenses which can be electrically deformed for focusing images of the display screen (5) at required distances.

17. The equipment for examining, diagnosing or aiding the diagnosis and therapy of functional vision problems according to claim 1, **characterized in that** the lens elements (6) are formed by several optical lenses which can be moved axially with respect to one another for focusing images of the display screen (5) at required distances.

18. The equipment for examining, diagnosing or aiding the diagnosis and therapy of functional vision problems according to claim 14, **characterized in that** the lens elements (6) are assembled in the cartridges (19) by means of elastic elements (22) which are able to tilt, which allow orientating said lens elements (6) with respect to the user's eyes by means of a carriage (23) which can be moved transversally inside the respective casing (4).

19. The equipment for examining, diagnosing or aiding the diagnosis and therapy of functional vision problems according to claim 14, **characterized in that** the lens elements (6) are assembled in the cartridges (19) by means of elastic elements (22) which are able to tilt, which allow orienting said lens elements (6) with respect to the user's eyes by means of formations integral with the casing (4), said casing (4) being able to be moved vertically and/or horizontally.

20. The equipment for examining, diagnosing or aiding the diagnosis and therapy of functional vision problems according to claim 9, **characterized in that** closure gates (21) are arranged with respect to the side openings (17) allowing the selective blocking thereof.

21. The equipment for examining, diagnosing or aiding the diagnosis and therapy of functional vision problems according to claim 9, **characterized in that** closures made of electro-optical material susceptible to transparency variation which allow selectively blocking the passage of light through the side openings (17) are arranged with respect to said side openings (17).

22. A method for examining, diagnosing or aiding the diagnosis and therapy of functional vision problems with the equipment of claim 1, **characterized in that** images are shown on the display screens (5) arranged in front of the user's eyes, maintaining a verbal or visual communication with the user through an electronic unit (10), and while the user's eyes view said images, parameters such as the movement of each eye, the point of convergence of the gaze, the pupil diameter, the distance of the focal plane and the lens curvature are measured or calculated for determining, from said measured or calculated parameters, eye performance patterns, which are compared with pre-established patterns defining normal vision for obtaining the functional vision problems and the eyes are subjected to exercises by means of projecting images on the screens (5) which allow improving the functional problems detected.

23. The method for examining, diagnosing or aiding the diagnosis and therapy of functional vision problems according to claim 22, **characterized in that** the image of the user's eyes is captured by means of the cameras (7) and the direction of gaze of each eye, the point of convergence of a user's gaze and the distance of the point of convergence to the user are calculated from such image in the electronic unit (10), controlling the lens elements (6) so that they adapt their focal length according to the actual depth corresponding to the distance of the point of convergence of the user's gaze to the user.

24. The method for examining, diagnosing or aiding the diagnosis and therapy of functional vision problems according to claim 22, **characterized in that** each image projected on the display screens (5) is coded according to actual depths and a specific blurriness, which increases progressively in increasingly farther planes, is assigned to the contents of each plane of the image depicted on the plane of the display according to its virtual depth or distance to the user by means of the electronic unit (10).
